# EUROPEAN PATENT APPLICATION

(11) **EP 4 435 043 A1**
(43) Date of publication of application: **25.09.2024**
(21) Application number: 23892062.3
(22) Date of filing: 17.11.2023
(51) Int. Cl.: C08J 11/24, C08J 11/16, C07D 317/38, C07D 317/36, C08J 11/18, C08J 11/28

(54) **RECYLABLE CYCLIC CARBONATE COMPOSITION AND METHOD FOR MANUFACTURING SAME**

(30) Priority: 18.11.2022 KR 20220155347; 20.04.2023 KR 20230052249; 16.11.2023 KR 20230159224; 16.11.2023 KR 20230159225
(71) Applicant: LG Chem, Ltd., Yeongdeungpo-gu Seoul 07336 (KR)
(72) Inventor: LEE, Hyunyoung, Daejeon 34122 (KR); PARK, Jun Beom, Daejeon 34122 (KR); KIM, Hyun Cheol, Daejeon 34122 (KR)
(74) Representative: Goddar, Heinz J.
(86) International application number: PCT/KR2023/018535
(87) International publication number: WO 2024/106999

(57) **Abstract**

The present invention relates to a recycled cyclic carbonate composition, comprising an alkylene carbonate, wherein the recycled cyclic carbonate composition further comprises a phenolic compound whose mass ratio measured by ¹H NMR is 5 mass% or less relative to a total mass of the composition, and wherein the recycled cyclic carbonate composition is a recovered product from a polycarbonate-based resin, and a method for preparing the same.

## Description

### [TECHNICAL FIELD]

### CROSS-REFERENCE TO RELATED APPLICATION(S)

This application claims the benefit of Korean Patent Application No. 10-2022-0155347 filed on November 18, 2022, Korean Patent Application No. 10-2023-0052249 filed on April 20, 2023, Korean Patent Application No. 10-2023-0159224 filed on November 16, 2023, and Korean Patent Application No. 10-2023-0159225 filed on November 16, 2023 in the Korean Intellectual Property Office, the disclosures of which are incorporated herein by reference in their entirety.

The present invention relates to a recycled cyclic carbonate composition recovered through recycling by chemical decomposition of a polycarbonate-based resin, and a preparation method of the same.

### [BACKGROUND]

Polycarbonate is a thermoplastic polymer and is a plastic having excellent characteristics such as excellent transparency, superior ductility, and relatively low production costs.

Although polycarbonate is widely used for various purposes, environmental and health concerns during waste treatment have been continuously raised.

Currently, a physical recycling method is carried out, but in this case, a problem accompanying the deterioration of the quality occurs, and thus, research on the chemical recycling of polycarbonate is underway.

Chemical decomposition of a polycarbonate refers to obtaining an aromatic diol compound (e.g., bisphenol A: BPA) and a carbonate compound (e.g., diethyl carbonate) which are monomers constituting polycarbonate through decomposition of a polycarbonate.

For such a chemical decomposition, thermal decomposition, hydrolysis, and alcohol decomposition are typically known. Among them, the most common method is alcohol decomposition, but in the case of methanol decomposition, there is a problem that methanol is used which is harmful to the human body, and in the case of ethanol, there is a problem that high temperature and high pressure conditions are required and the yield is not high.

Meanwhile, cyclic carbonates such as ethylene carbonate are useful as solvents for high molecular weight polymers and synthetic fuels for carbonate esters, and can also be applied as liquid electrolyte organic solvents for electric vehicle batteries. Conventionally, ethylene carbonate has been prepared through industrial synthesis using ethylene oxide and carbon dioxide, but there is a need to develop more environmentally friendly methods.

Therefore, as mentioned above, there is a growing need to develop a method for preparing industrially useful cyclic carbonates by chemical decomposition of a polycarbonate, taking advantage of the fact that carbonate compounds can be obtained through chemical decomposition of a polycarbonate.

### [DETAILED DESCRIPTION OF THE INVENTION]

### [Technical Problem]

It is an object of the present invention to provide a recycled cyclic carbonate composition and a preparation method of the same, in which a cyclic carbonate compound recovered through recycling by chemical decomposition of a polycarbonate-based resin can be secured with high purity.

### [Technical Solution]

In order to achieve the above object, provided herein is a recycled cyclic carbonate composition, comprising an alkylene carbonate, wherein the recycled cyclic carbonate composition further comprises a phenolic compound whose mass ratio measured by ¹H NMR is 5 mass% or less relative to a total mass of the composition, and wherein the recycled cyclic carbonate composition is a recovered product from a polycarbonate-based resin.

Also provided herein is a method for preparing a recycled cyclic carbonate composition, the method comprising the steps of: adding a polycarbonate-based resin to an organic solvent to prepare a mixed solution; adding an alkylene glycol and an inorganic base catalyst to the mixed solution to depolymerize the polycarbonate-based resin; removing an aromatic diol compound, an organic solvent, and an alkylene glycol from the depolymerized solution; and recovering an alkylene carbonate from the depolymerized solution from which the aromatic diol compound, the organic solvent, and the alkylene glycol have been removed.

Below, a recycled cyclic carbonate composition and a method for preparing the same according to specific embodiments of the present invention will be described in more detail.

Unless explicitly stated herein, the technical terms used herein are for the purpose of describing specific embodiments only and is not intended to limit the scope of the invention.

The singular forms "a," "an" and "the" used herein are intended to include plural forms, unless the context clearly indicates otherwise.

It should be understood that the terms "comprise," "include", "have", etc. are used herein to specify the presence of stated feature, region, integer, step, action, element and/or component, but do not preclude the presence or addition of one or more other feature, region, integer, step, action, element, component and/or group.

Further, the terms including ordinal numbers such as "a first", "a second", etc. are used only for the purpose of distinguishing one component from another component, and are not limited by the ordinal numbers. For instance, a first component may be referred to as a second component, or similarly, the second component may be referred to as the first component, without departing from the scope of the present invention.

As used herein, the term "substituted or unsubstituted" means being unsubstituted or substituted with one or more substituent groups selected from the group consisting of deuterium; a halogen group; a cyano group; a nitro group; a hydroxy group; a carbonyl group; an ester group; an imide group; an amino group; a primary amino group; a carboxy group; a sulfonic acid group; a sulfonamide group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkoxysilylalkyl group; an arylphosphine group; or a heterocyclic group containing at least one of N, O and S atoms, or being unsubstituted or substituted with a substituent linking two or more substituent groups of the substituents illustrated above. For example, "a substituent linking two or more substituents" may be a biphenyl group. In other words, a biphenyl group may be an aryl group, or it may also be interpreted as a substituent linking two phenyl groups.

As used herein, the alkyl group is a monovalent functional group derived from an alkane, and may be a straight-chain or a branched-chain. The number of carbon atoms of the straight chain alkyl group is not particularly limited, but is preferably 1 to 20. Also, the number of carbon atoms of the branched chain alkyl group is 3 to 20. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 2-methylpentyl, 4-methylhexyl, 5-methylhexyl, 2,6-dimethylheptane-4-yl and the like, but are not limited thereto. The alkyl group may be substituted or unsubstituted, and when substituted, examples of the substituent are the same as described above.

As used herein, the alkylene group is a bivalent functional group derived from alkane, and the description of the aryl group as defined above can be applied except that these are divalent functional groups. For example, the linear or branched alkylene group may include a methylene group, an ethylene group, a propylene group, an isobutylene group, a sec-butylene group, a tert-butylene group, a pentylene group, a hexylene group, and the like. The alkylene group can be substituted or unsubstituted, and when substituted, examples of the substituent are the same as described above.

As used herein, "one or more" means, for example, "1, 2, 3, 4 or 5, particularly 1, 2, 3 or 4, more particularly 1, 2 or 3, and even more particularly 1 or 2".

### 1. Recycled cyclic carbonate composition

According to one embodiment of the present invention, there can be provided a recycled cyclic carbonate composition, comprising an alkylene carbonate, wherein the recycled cyclic carbonate composition further comprises a phenolic compound whose mass ratio measured by ¹H NMR is 5 mass% or less relative to a total mass of the composition, and wherein the recycled cyclic carbonate composition is a recovered product from a polycarbonate-based resin.

The present inventors have found through experiments that although the recycled cyclic carbonate composition of the one embodiment was recovered through recycling by chemical decomposition of the polycarbonate-based resin, the purity is high and the proportion of impurities is remarkably reduced at the level of a newly synthesized alkylene carbonate, whereby excellent physical properties can be realized when applied to a variety of applications (for example, solvents for high molecular weight polymers, synthetic fuels for carbonate esters, liquid electrolyte organic solvents for electric vehicle batteries, etc.) using the same, and completed the present invention.

That is, the present invention may have technical features and advantages that a composition comprising the alkylene carbonate can be obtained with high purity through recycling by chemical decomposition of a polycarbonate-based resin.

Specifically, the recycled cyclic carbonate composition according to the one embodiment is characterized by being recovered from a polycarbonate-based resin. That is, this means that as a result of proceeding the recovery from the polycarbonate-based resin in order to obtain the recycled cyclic carbonate composition according to the one embodiment, the recycled cyclic carbonate composition containing an alkylene carbonate is obtained together.

The polycarbonate-based resin is meant to include both a homopolymer and a copolymer containing a polycarbonate repeating unit, and collectively refers to a reaction product obtained through a polymerization reaction or a copolymerization reaction of a monomer containing an alkylene carbonate and a carbonate precursor. When it contains one type of carbonate repeating unit obtained by using only one type of alkylene carbonate and one type of carbonate precursor, a homopolymer can be synthesized. In addition, when one type of alkylene carbonate and two or more types of carbonate precursors are used as the monomer, or two or more types of alkylene carbonates and one type of carbonate precursor are used, or one or more types of other diols is used in addition to the one type of alkylene carbonate and the one type of carbonate precursor to contain two or more types of carbonates, a copolymer can be synthesized. The homopolymer or copolymer can include all of low-molecular compounds, oligomers, and polymers depending on the molecular weight range.

Further, the recycled cyclic carbonate composition according the one embodiment may include an alkylene carbonate. Specific examples of the an alkylene carbonate include ethylene carbonate, propylene carbonate, or mixtures thereof.

The alkylene carbonate is characterized by being recovered from the polycarbonate-based resin used for recovering the recycled cyclic carbonate composition. That is, this means that as a result of performing the recovery from the polycarbonate-based resin in order to obtain the recycled cyclic carbonate composition according to the one embodiment, an alkylene carbonate is also obtained together. Therefore, apart from the recovery from the polycarbonate-based resin in order to prepare the recycled cyclic carbonate composition according to the one embodiment, the case where a novel alkylene carbonate is added from the outside is not included in the category of the alkylene carbonate of the present invention.

Specifically, "recovered from the polycarbonate-based resin" means being obtained through a depolymerization reaction of the polycarbonate-based resin. The depolymerization reaction can be carried out under acidic, neutral or basic conditions, and particularly, the depolymerization reaction can be carried out under basic (alkaline) conditions. Particularly, the depolymerization reaction can be preferably carried out in the presence of an alcohol, as will be described later.

Meanwhile, in the recycled cyclic carbonate composition, a mass ratio of the alkylene carbonate measured by ¹H NMR may be 95 mass% to 98.5 mass%, or 95.11 mass% to 98.46 mass%, or 95.5 mass% to 98.5 mass%, or 96 mass% to 98.5 mass%, or 97 mass% to 98.5 mass%, or 98 mass% to 98.5 mass% relative to the total mass of the composition.

Examples of the method for measuring the mass ratio of the alkylene carbonate measured by ¹H NMR are not particularly limited, and for example, the mass ratio of the alkylene carbonate can be determined through the following Equation 1. Ethylene carbonate(EC) mass ratio (mass%) = (Mol.eqEC × MWEC)/[(Mol.eqPTBP × MWPTBP) + (Mol.eqMHE-BPA × MWMHE-BPA) + (Mol.eqBPA × MWBPA) + (Mol.eqEG × MWEG) + (Mol.eqEC × MWEC)] × 100
wherein, in Equation 1, MW_{EC} is the molecular weight of ethylene carbonate(EC), MW_{EG} is the molecular weight of ethylene glycol(EG), MW_{BPA} is the molecular weight of bisphenol A (BPA), MW_{PTBP} is the molecular weight of 4-tert-butylphenol(PTBP), MW_{MHE-BPA} is the molecular weight of monohydroxyethyl-bisphenol A (MHE-BPA),
Mol.eq_{EC} is I_{EC}/H_{EC},
I_{EC} is (S_{EC})/[(S_{PTBP})+(S_{MHE-BPA})+(S_{BPA})+(S_{EG})+(S_{EC})],
S_{EC} is the peak integral value at 4.48 ppm (s, 2CH₂) of ethylene carbonate(EC), S_{EG} is the peak integral value at 3.42 ppm (d, 2CH₂) of ethylene glycol(EG), S_{BPA} is the peak integral value at 6.67 ppm (d, 4CH) of bisphenol A(BPA), S_{PTBP} is the peak integral value at 6.60 ppm (d, 2CH) of 4-tert-butylphenol(PTBP), S_{MHE-BPA} is the peak integral value at 6.85 ppm (d, 2CH) of monohydroxyethyl-bisphenol A(MHE-BPA), H_{EC} is the number of H in ethylene carbonate(EC), Mol.eq_{EG} is I_{EG}/H_{EG},
I_{EG} is (S_{EG})/[(S_{PTBP})+(S_{MHE-BPA})+(S_{BPA})+(S_{EG})+(S_{EC})],
H_{EG} is the number of H in ethylene glycol(EG),
Mol.eq_{BPA} is I_{BPA}/H_{BPA},
I_{BPA} is (S_{BPA})/[(S_{PTBP})+(S_{MHE-BPA})+(S_{BPA})+(S_{EG})+(S_{EC})],
H_{BPA} is the number of H in bisphenol A(BPA),
Mol.eq_{PTBP} is I_{PTBP}/H_{PTBP},
I_{PTBP} is (S_{PTBP})/[(S_{PTBP})+(S_{MHE-BPA})+(S_{BPA})+(S_{EG})+(S_{EC})],
H_{PTBP} is the number of H in 4-tert-butylphenol(PTBP),
Mol eq_{MHE-BPA} i s I_{MHE-BPA}/H_{MHE-BPA},
I_{MHE-BPA} is (S_{MHE-BPA})/[(S_{PTBP})+(S_{MHE-BPA})+(S_{BPA})+(S_{EG})+(S_{EC})],
H_{MHE-BPA} is the number of H in monohydroxyethyl-bisphenol A(MHE-BPA).

As for the specific methods, conditions, equipment, etc. of the ¹H NMR, various well-known contents can be applied without limitation.

As described above, the mass ratio of the alkylene carbonate measured by ¹H NMR in the recycled cyclic carbonate composition of the one embodiment is extremely increased to 95 mass% to 98.5 mass% relative to the total mass of the composition, whereby the proportion of impurities other than alkylene carbonate, which is the main recovery target material, is remarkably reduced, thereby capable of realizing excellent physical properties when applied to a variety of applications (for example, solvents for high molecular weight polymers, synthetic fuels for carbonate esters, liquid electrolyte organic solvents for electric vehicle batteries, etc.) using the same.

Looking more specifically at the above-mentioned impurities, the recycled cyclic carbonate composition of the one embodiment further comprises a phenolic compound, wherein the mass ratio of the phenolic compound measured by ¹H NMR may be 5 mass% or less, or 4.5 mass% or less, or 4.35 mass% or less, or 4 mass% or less, or 3 mass% or less, or 2 mass% or less, or 1.5 mass% or less, or 1.2 mass% or less, or 0.01 mass% or more, or 1.1 mass% or more relative to a total mass of the composition. By combining the upper limit numerical range and the lower limit numerical range, the numerical range from the lower limit to the upper limit can also be satisfied.

As one example of the numerical range between the lower limit and the upper limit, the mass ratio of the phenolic compound measured by ¹H NMR in the recycled cyclic carbonate composition may be 0.01 mass% to 5 mass% relative to the total mass of the composition.

Examples of the method for measuring the mass ratio of the phenolic compound measured by ¹H NMR are not particularly limited, and for example, the mass ratio of bisphenol A is determined through the following Equation 3, the mass ratio of 4-tert-butylphenol through the following Equation 4, and the mass ratio of monohydroxyethyl-bisphenol A through the following Equation 5, respectively, and then can be determined through the total of these. BPA mass ratio (mass%) = (Mol.eqBPA X MWBPA)/[(Mol.eqPTBP X MWPTBP) + (Mol.eqMHE-BPA X MWMHE-BPA) + (Mol.eqBPA X MWBPA) + (Mol.eqEG X MWEG) + (Mol.eqEC × MWEC)] X 100 PTBP mass ratio (mass%) = (Mol.eqPTBP X MWPTBP)/[(Mol.eqPTBP X MWPTBP) + (Mol.eqMHE-BPA X MWMHE-BPA) + (Mol.eqBPA X MWBPA) + (Mol.eqEG X MWEG) + (Mol.eqEC X MWEC)] X 100 MHE-BPA mass ratio (mass%) = (Mol.eqMHE-BPA X MWMHE-BPA)/[(Mol.eqPTBP X MWPTBP) + (Mol.eqMHE-BPA X MWMHE-BPA) + (Mol.eqBPA X MWBPA) + (Mol.eqEG X MWEG) + (Mol.eqEC × MWEC)] X 100

The description of each calculation formula in Equations 3 to 5 includes all the contents described above in Equation 1.

The phenolic compound may include one or more compounds selected from the group consisting of bisphenol A, 4-tert-butylphenol, and monohydroxyethyl-bisphenol A. That is, the phenolic compound may include one type of bisphenol A, one type of 4-tert-butylphenol, one type of monohydroxyethyl-bisphenol A, or a mixture of two or more types thereof.

More specifically, in the recycled cyclic carbonate composition of the one embodiment, the mass ratio of the phenolic compound relative to 100 parts by mass of the alkylene carbonate may be 0.01 parts by mass to 5 parts by mass, or 0.01 parts by mass to 4.5 parts by mass, or 0.01 parts by mass to 4.35 parts by mass, or 0.01 parts by mass to 4 parts by mass, or 0.01 parts by mass to 3 parts by mass, or 0.01 parts by mass to 2 parts by mass, or 0.01 parts by mass to 1.5 parts by mass, or 0.01 parts by mass to 1.2 parts by mass.

In this manner, in the recycled cyclic carbonate composition of the one embodiment, the mass ratio of the phenolic compound measured by ¹H NMR is extremely reduced to 5 mass% or less relative to the total mass of the composition, whereby the proportion of impurities other than the alkylene carbonate, which is the main recovery target material, is remarkably reduced, thereby capable of realizing excellent physical properties when applied to a variety of applications (for example, solvents for high molecular weight polymers, synthetic fuels for carbonate esters, liquid electrolyte organic solvents for electric vehicle batteries, etc.) using the same.

Conversely, if the mass ratio of the phenolic compound is excessively increased, the viscosity of the alkylene carbonate may become high and the solubility of the salt may decrease. This may cause technical problems of decreasing the ionic viscosity decreases and lowering the chemical stability when applied to batteries.

In addition, the recycled cyclic carbonate composition of the one embodiment further comprises an alkylene glycol, wherein the mass ratio of the alkylene glycol measured by ¹H NMR may be 0.1 mass% to 3 mass%, or 0.38 mass% to 2.49 mass%, or 0.1 mass% to 2.5 mass%, or 0.1 mass% to 2 mass%, or 0.1 mass% to 1 mass%, or 0.1 mass% to 0.5 mass%, or 0.1 mass% to 0.4 mass% relative to the total mass of the composition.

Examples of the method for measuring the mass ratio of the alkylene glycol measured by ¹H NMR are not particularly limited, and for example, the mass ratio of ethylene glycol can be obtained through the following Equation 2. EG mass ratio (mass%) = (Mol.eqEG X MWEG)/[(Mol.eqPTBP X MWPTBP) + (Mol.eqMHE-BPA X MWMHE-BPA) + (Mol.eqBPA X MWBPA) + (Mol.eqEG X MWEG) + (Mol.eqEC X MWEC)] X 100 wherein, the description of each calculation formula in Equation 2 includes all the contents described above in Equation 1.

The alkylene glycol may include ethylene glycol.

More specifically, in the recycled cyclic carbonate composition of the one embodiment, the mass ratio of the alkylene glycol relative to 100 parts by mass of the alkylene carbonate is 0.1 part by mass to 3 parts by mass, or 0.1 part by mass to 2.6 parts by mass, or 0.1 part by mass to 2.5 parts by mass, or 0.1 part by mass to 2 parts by mass, or 0.1 part by mass to 1 part by mass, or 0.1 part by mass to 0.5 part by mass, or 0.1 part by mass to 0.4 part by mass.

In this way, the mass ratio of the alkylene glycol measured by ¹H NMR in the recycled cyclic carbonate composition of one embodiment is extremely reduced to 0.1% by mass to 3% by mass relative to the total mass of the composition, whereby the proportion of impurities other than the alkylene carbonate, which is the main recovery target material, is remarkably reduced, thereby capable of realizing excellent physical properties when applied to a variety of applications (for example, solvents for high molecular weight polymers, synthetic fuels for carbonate esters, liquid electrolyte organic solvents for electric vehicle batteries, etc.) using the same.

Conversely, if the mass ratio of the alkylene glycol is excessively increased, it is not easy to manage the moisture content in the alkylene carbonate, which may lead to technical problems such as leakage current.

In addition, the recycled cyclic carbonate composition of the one embodiment further comprises a phenolic compound and an alkylene glycol, wherein a mass ratio of the phenolic compound relative to 100 parts by mass of the alkylene glycol may be 30 parts by mass to 1000 parts by mass, or 50 parts by mass to 1000 parts by mass, or 100 parts by mass to 1000 parts by mass, or 300 parts by mass to 1000 parts by mass, or 30 parts by mass to 950 parts by mass, or 30 parts by mass to 900 parts by mass, or 30 parts by mass to 860 parts by mass, or 30 parts by mass to 500 parts by mass.

Meanwhile, a calcium ion weight ratio in the recycled cyclic carbonate composition measured using ion chromatography may be 0.05 ppm to 0.4 ppm, or 0.1 ppm to 0.3 ppm, or 0.2 ppm to 0.3 ppm, or 0.1 ppm to 0.2 ppm, based on 1 g of the composition.

Further, a chloride ion weight ratio in the recycled cyclic carbonate composition measured using ion chromatography 5.1 ppm to 20 ppm, or 7 ppm to 20 ppm, or 10 ppm to 20 ppm, or 10 ppm to 15 ppm, or 10 ppm to 12 ppm, based on 1 g of the composition.

In addition, the total ion concentration in the recycled cyclic carbonate composition measured using ion chromatography may be 300 ppm or less, or 200 ppm or less, or 100 ppm or less, or 50 ppm or less, or 40 ppm or less, or 30 ppm or less, or 20 ppm or less, or 15 ppm or less, or 10 ppm or less, or 1 ppm or less based on 1g of the composition. By combining the upper limit numerical range and the lower limit numerical range, the numerical range from the lower limit to the upper limit can also be satisfied. As an example of the numerical range between the lower limit and the upper limit, the total ion concentration in the recycled cyclic carbonate composition measured using ion chromatography may be 1 ppm to 300 ppm based on 1 g of the composition.

The sum of the total ion concentration in the recycled cyclic carbonate composition measured using ion chromatography is reduced to 300 ppm or less, or 200 ppm or less, or 100 ppm or less, or 50 ppm or less, or 40 ppm or less, or 30 ppm or less, or 20 ppm or less, or 15 ppm or less, or 10 ppm or less based on 1g of the composition, whereby the recycled cyclic carbonate composition can be used as an electrolyte to realize excellent effects.

On the other hand, if the sum of the total ion concentration in the recycled cyclic carbonate composition measured using ion chromatography increases excessively based on 1 g of the composition, ionic impurities may cause technical problems such as electrode corrosion.

Examples of the method for measuring the ionic impurity weight ratio of the recycled cyclic carbonate composition of the one embodiment is not particularly limited, and an ion chromatography(IC) analysis can be used. As for the specific methods, conditions, equipment, etc. of the IC, various well-known contents can be applied without limitation. However, in one example, measurement can be made under the following conditions: ① Main Column: IonPac AS18 analytical (4X250 mm) ② Guard Column: Ion AG18 guard (4X50 mm) (3) Type of Eluent: KOH (30.5mM) ④ Eluent flow rate: 1mL/min (5) Detector: Suppressed Conductivity Detector ⑥ SRS Current: 76mA ⑦ Injection volume: 20*µ*ℓ ⑧ Isocratic/Gradient conditions: isocratic conditions.

As described above, the weight ratio of ionic impurities other than alkylene carbonate, which is the main recovery target material in the recycled cyclic carbonate composition of one embodiment, is remarkably reduced, thereby capable of realizing excellent physical properties.

Meanwhile, in the recycled cyclic carbonate composition, a peak area ratio of the alkylene carbonate measured by GC-MS may be 87 area% to 99.99 area%, or 90 area% to 99.99 area%, or 95 area% to 99.99 area%, or 97 area% to 99.99 area% relative to a total peak area. As described above, in the recycled cyclic carbonate composition of one embodiment, the peak area ratio of the alkylene carbonate measured by GC-MS is remarkably increased from 87 area % to 99.99 area % relative to the total peak area, whereby the proportion of impurities other than alkylene carbonate, which is the main recovery target material, is remarkably reduced, thereby capable of realizing excellent physical properties when applied to a variety of applications (for example, solvents for high molecular weight polymers, synthetic fuels for carbonate esters, liquid electrolyte organic solvents for electric vehicle batteries, etc.) using the same.

Further, in the recycled cyclic carbonate composition, the peak area ratio of the phenolic compounds measured by GC-MS may be 0.01 area% to 12 area%, or 0.01 area% to 11 area%, or 0.01 area% to 10 area%, or 0.01 area % to 5 area %, or 0.01 area % to 4 area %, or 0.01 area % to 3 area % relative to the total peak area. The phenolic compound may include one or more compounds selected from the group consisting of bisphenol A, 4-tert-butylphenol, and monohydroxyethyl-bisphenol A. That is, the phenolic compound may include one type of bisphenol A, one type of 4-tert-butylphenol, one type of monohydroxyethyl-bisphenol A, or a mixture of two or more types thereof.

Examples of the method for measuring the peak area ratio of the phenolic compounds measured by GC-MS are not particularly limited, and for example, the peak area ratio of bisphenol A, the peak area ratio of 4-tert-butylphenol, and the peak area ratio of monohydroxyethyl-bisphenol A are obtained, respectively, and then obtained by the total of these.

More specifically, the ethylene carbonate(EC) may show a peak at a retention time of 8 minutes to 11 minutes, bisphenol A (BPA) may show a peak at a retention time of 24 minutes to 26 minutes, 4-tert-butylphenol (PTBP) may show a peak at a retention time of 14 minutes to 16 minutes, and monohydroxyethyl-bisphenol A (MHE-BPA) may show a peak at a retention time of 26 minutes to 28 minutes.

As for the specific methods, conditions, equipment, etc. of the gas chromatography mass spectrometry (GC-MS), various well-known contents can be applied without limitation. However, in one example, measurement can be made under the following conditions: ① Column: HP-5MS(L:30m, ID:0.25mm, film:0.25µm) ② Injection volume: 0.2*µ*ℓ (3) Inlet, Temp.: 300 °C, spilt ratio: 20:1 ④ Column flow: (He) 1ml/min⑤ Oven temp.: 50°C/5min-10°C/min-320°C/15min (Total 47min) ⑥ Detector Temp.:300°C ⑦ GC Model: Agilent 7890.

As described above, the proportion of impurities other than alkylene carbonate, which is the main recovery target material in the recycled cyclic carbonate composition of one embodiment, is remarkably reduced, thereby capable of realizing excellent physical properties when applied to a variety of applications (for example, solvents for high molecular weight polymers, synthetic fuels for carbonate esters, liquid electrolyte organic solvents for electric vehicle batteries, etc.) using the same.

The recycled cyclic carbonate composition of the one embodiment may further include small amounts of other additives and solvents. Specific types of the additives or solvents are not particularly limited, and various materials widely used in the process of recovering the alkylene carbonate by a depolymerization of the polycarbonate-based resin can be applied without limitation.

The recycled cyclic carbonate composition of the one embodiment can be obtained by a method for preparing the recycled cyclic carbonate composition, which will be described later. That is, the recycled cyclic carbonate composition of one embodiment corresponds to the result obtained through various processes of filtration, purification, washing, and drying in order to secure only the alkylene carbonate, which is the main recovery target material, with high purity after the depolymerization reaction of the polycarbonate-based resin.

### 2. Method for preparing recycled cyclic carbonate composition

According to another embodiment of the invention, there can be provided a method for preparing the recycled cyclic carbonate composition, the method comprising the steps of: adding a polycarbonate-based resin to an organic solvent to prepare a mixed solution; adding an alkylene glycol and an inorganic base catalyst to the mixed solution to depolymerize the polycarbonate-based resin; removing an aromatic diol compound, an organic solvent, and an alkylene glycol from the depolymerized solution; and recovering an alkylene carbonate from the depolymerized solution from which the aromatic diol compound, the organic solvent, and the alkylene glycol have been removed.

The present inventors have found through experiments that although the recycled cyclic carbonate composition of the other embodiments was recovered through recycling by chemical decomposition of the polycarbonate-based resin, it has a high purity at the level of a newly synthesized alkylene carbonate, thereby capable of realizing excellent physical properties, and securing with high yield, and completed the present invention.

Specifically, according to the present invention, a polycarbonate is decomposed to an alkylene glycol under mild conditions, thereby being able to stably obtain an alkylene glycol, which is a monomer having high purity.

A method for preparing the recycled cyclic carbonate composition according to the other embodiment may comprise the steps of: adding a polycarbonate-based resin to an organic solvent to prepare a mixed solution, and adding an alkylene glycol and an inorganic base catalyst to the mixed solution to depolymerize the polycarbonate-based resin.

The polycarbonate-based resin is meant to include both a homopolymer and a copolymer containing a polycarbonate repeating unit, and collectively refers to a reaction product obtained through a polymerization reaction or a copolymerization reaction of a monomer containing an alkylene carbonate and a carbonate precursor. When it contains one type of carbonate repeating unit obtained by using only one type of alkylene carbonate and one type of carbonate precursor, a homopolymer can be synthesized. In addition, when one type of alkylene carbonate and two or more types of carbonate precursors are used as the monomer, or two or more types of alkylene carbonates and one type of carbonate precursor are used, or one or more types of other diols are used in addition to the one type of alkylene carbonate and the one type of carbonate precursor to contain two or more types of carbonates, a copolymer can be synthesized. The homopolymer or copolymer can include all of low-molecular compounds, oligomers, and polymers depending on the molecular weight range.

The polycarbonate-based resin can be applied regardless of various forms and types, such as a novel polycarbonate produced through synthesis, a recycled polycarbonate produced through a recycling process, or polycarbonate waste.

In the present invention, specific examples of the alkylene glycol are not particularly limited, and various known alkylene glycols can be used without limitation. Examples thereof include ethylene glycol or propylene glycol.

Meanwhile, the organic solvent may include one or more solvents selected from the group consisting of tetrahydrofuran, methylene chloride, chloroform, dimethyl carbonate, ethylmethyl carbonate, diethyl carbonate and dipropylcarbonate. That is, the organic solvent may include tetrahydrofuran, methylene chloride, chloroform, dimethyl carbonate, ethylmethyl carbonate, diethyl carbonate, dipropyl carbonate, or a mixture of two or more thereof.

Specifically, when methylene chloride is used as an organic solvent, there is an advantage that the dissolution properties in polycarbonate can be improved and the reactivity can be improved.

The weight ratio of the alkylene glycol, the organic solvent, and the polycarbonate-based resin is not particularly limited, however, for example, the weight ratio between the alkylene glycol: polycarbonate-based resin may be 10:1 to 1:10, or 1:1 to 1:10, or 1:1 to 1:5, or 1:1 to 1:2, or 1:1.1 to 1:2.

Further, the weight ratio between the organic solvent and the polycarbonate-based resin may be 10:1 to 1:10, or 10:1 to 1:1, or 10:1 to 2:1, or 10:1 to 5:1, or 10:1 to 6:1, or 10:1 to 7:1, or 10:1 to 8:1, or 9:1 to 8:1, or 9:1 to 5:1, or 8:1 to 5:1, or 7:1 to 5:1, or 6:1 to 5:1.

Further, the weight ratio between organic solvent and the alkylene glycol may be 20:1 to 1:20, or 1:1 to 20:1, or 5:1 to 20:1, or 5:1 to 15:1, or 5:1 to 11:1.

Specifically, by mixing the alkylene glycol and the organic solvent within the above range, there is an advantage that the depolymerization reaction of the polymer can proceed at a desired level.

The catalyst may be added in an amount of 0.1 to 10 parts by weight, or 0.1 to 5 parts by weight, based on 100 parts by weight of the polycarbonate-based resin. Specifically, by containing the catalyst in the above content range, there is an advantage that economical catalytic reactions can proceed.

The catalyst may be an inorganic base catalyst. The inorganic base catalyst means a nonionic inorganic compound having basicity, and specifically, it may be sodium hydroxide(NaOH) or potassium hydroxide(KOH), preferably sodium hydroxide(NaOH), without being limited thereto. The use of the inorganic base catalyst allows the decomposition reaction to proceed under mild conditions, which is economically advantageous.

Meanwhile, the step of adding an alkylene glycol and an inorganic base catalyst to the mixed solution to depolymerize the polycarbonate-based resin may be stirring at 20°C to 100°C, or 50°C to 100°C, or 60°C to 100°C, or 70°C to 100°C for 1 hour to 24 hours, or 1 hour to 12 hours, or 1 hour to 8 hours.

Specifically, the conditions are mild process conditions as compared to the conventional pressurizing/high temperature process, and by performing stirring under the above conditions, the process can be performed in a mild process as compared to the pressurizing/high temperature process. In particular, when stirring at 70°C to 100°C for 1 to 12 hours, there is an advantage of obtaining the most efficient results in terms of reproducibility and acceptability.

Meanwhile, the method for preparing the recycled cyclic carbonate composition according to the other embodiment may comprise a step of removing an aromatic diol compound, an organic solvent, and an alkylene glycol from the depolymerized solution. Through the step of removing an aromatic diol compound, an organic solvent, and an alkylene glycol from the depolymerized solution, an alkylene carbonate in which the organic solvent, alkylene glycol, and aromatic diol compound are all separated and remain, can be obtained as the main product.

The step of removing an aromatic diol compound, an organic solvent, and an alkylene glycol from the depolymerized solution may comprise the steps of: removing the aromatic diol compound from the depolymerized solution; removing the organic solvent from the depolymerized solution; and removing the alkylene glycol from the depolymerized solution.

The order of the step of removing the aromatic diol compound from the depolymerized solution; the step of removing the organic solvent from the depolymerized solution; and the step of removing the alkylene glycol from the depolymerized solution, is not particularly limited, however, for example, the step of removing the aromatic diol compound from the depolymerized solution; the step of removing the organic solvent from the depolymerized solution; and the step of removing the alkylene glycol from the depolymerized solution can proceed in this order.

More specifically, the step of removing the aromatic diol compound from the depolymerized solution may comprise the steps of: crystallizing the aromatic diol compound formed by the depolymerization; and filtering the aromatic diol compound crystals.

The aromatic diol compound is characterized in that it is recovered from the polycarbonate-based resin. That is, this means that as a result of performing the recovery from the polycarbonate-based resin in order to obtain the recycled cyclic carbonate composition of the other embodiment, the aromatic diol compound is obtained together.

Specifically, "recovered from the polycarbonate-based resin" means being obtained through a depolymerization reaction of the polycarbonate-based resin. The depolymerization reaction can be carried out under acidic, neutral or basic conditions, and particularly, the depolymerization reaction can proceed under basic (alkaline) conditions. Particularly, the depolymerization reaction can be preferably carried out in the presence of a glycol-based compound, as will be described later.

Specific examples of the aromatic diol compound include bis(4-hydroxyphenyl)methane, bis(4-hydroxyphenyl)ether, bis(4-hydroxyphenyl)sulfone, bis(4-hydroxyphenyl)sulfoxide, bis(4-hydroxyphenyl)sulfide, bis(4-hydroxyphenyl)ketone, 1,1-bis(4-hydroxyphenyl)ethane, 2,2-bis(4-hydroxyphenyl)propane (bisphenol A), 2,2-bis(4-hydroxyphenyl)butane, 1,1-bis(4-hydroxyphenyl)cyclohexane (bisphenol Z), 2,2-bis(4-hydroxy-3,5-dibromophenyl)propane, 2,2-bis(4-hydroxy-3,5-dichlorophenyl)propane, 2,2-bis(4-hydroxy-3-bromophenyl)propane, 2,2-bis(4-hydroxy-3-chlorophenyl)propane, 2,2-bis(4-hydroxy-3-methylphenyl)propane, 2,2-bis(4-hydroxy-3,5-dimethylphenyl)propane, 1,1-bis(4-hydroxyphenyl)-1-phenylethane, or a mixture of two or more thereof, and the like. Preferably, the aromatic diol compound may be 2,2-bis(4-hydroxyphenyl)propane (bisphenol A).

The step of crystallizing the aromatic diol compound formed by the depolymerization may proceed by adding the depolymerization solution to water. When the depolymerization solution is added to water, the aromatic diol compound contained in the depolymerized solution may be crystallized due to the difference in solubility. Crystallization conditions are not particularly limited, and various well-known crystallization conditions, methods, and equipment can be applied without limitation.

Among the aromatic diol compound, alkylene glycol, and alkylene carbonate contained in the depolymerized solution, the aromatic diol compound may form crystals, and the remaining materials may maintain a dissolved state. By filtering the aromatic diol compound crystallized at this time, the aromatic diol compound can be removed and an alkylene carbonate can be obtained.

In the step of filtering the aromatic diol compound crystals, the crystallized aromatic diol compound may be filtered under reduced pressure. Thereby, the aromatic diol compound contained in the depolymerized solution can be effectively removed. The filtration conditions are not particularly limited, and various well-known filtration conditions, methods, and equipment can be applied without limitation.

The step of removing the organic solvent from the depolymerized solution may include a step of distilling the organic solvent. The organic solvent may include one or more solvents selected from tetrahydrofuran, methylene chloride, chloroform, dimethyl carbonate, ethylmethyl carbonate, diethyl carbonate and dipropylcarbonate. That is, the organic solvent may include tetrahydrofuran, methylene chloride, chloroform, dimethyl carbonate, ethylmethyl carbonate, diethyl carbonate, dipropyl carbonate, or a mixture of two or more thereof. Specifically, when methylene chloride is used as an organic solvent, there is an advantage that the dissolution properties in polycarbonate can be improved and the reactivity can be improved.

The distillation temperature is adjusted to a temperature lower than the boiling point of the alkylene carbonate and higher than the boiling point of the organic solvent, so that the alkylene carbonate remains without being distilled, and the organic solvent can be removed by distillation. Other distillation conditions are not particularly limited, and various well-known distillation conditions, methods, and equipment can be applied without limitation.

Specifically, the step of distilling the organic solvent may proceed at a temperature of 40°C to 80°C, or 40°C to 70°C, or 40°C to 60°C, or 40°C to 50°C and under a pressure of 100 psi to 200 psi, or 120 psi to 180 psi, or 140 psi to 160 psi.

The step of removing the alkylene glycol from the depolymerized solution may comprise a step of adding the depolymerized solution to a solution separated into a water layer and an organic solvent layer, and removing an alkylene glycol dissolved in the water layer. The organic solvent may include one or more solvents selected from tetrahydrofuran, methylene chloride, chloroform, dimethyl carbonate, ethylmethyl carbonate, diethyl carbonate and dipropylcarbonate. That is, the organic solvent may include tetrahydrofuran, methylene chloride, chloroform, dimethyl carbonate, ethylmethyl carbonate, diethyl carbonate, dipropyl carbonate, or a mixture of two or more thereof. Specifically, when methylene chloride is used as an organic solvent, there is an advantage that the dissolution properties in polycarbonate can be improved and the reactivity can be improved.

Among the alkylene glycol and alkylene carbonate contained in the depolymerized solution, the alkylene glycol is relatively hydrophilic and can be dissolved in the water layer, and the alkylene carbonate can be dissolved in an organic solvent layer and separated into layers. At this time, the separated aqueous layer is removed to obtain an organic solvent layer, whereby the alkylene glycol can be removed to obtain an alkylene carbonate.

Meanwhile, the step of removing the aromatic diol compound, organic solvent, and alkylene glycol from the depolymerized solution may further comprise a step in which phenolic impurities derived from the aromatic diol compound formed by the depolymerization are removed with an adsorbent. The phenolic impurities may include one or more compounds selected from the group consisting of bisphenol A, 4-tert-butylphenol, and monohydroxyethyl-bisphenol A.

Examples of the adsorbent that can be used include activated carbon, charcoal, celite, or a mixture thereof. The activated carbon is a black carbon material having micropores produced by subjecting a raw material to a carbonization process at about 500°C and an activated carbon process at about 900°C, and examples thereof are not particularly limited, however, for example, various activated carbons such as plant-based, coal-based, petroleum-based, waste-based activated carbon can be applied without limitation depending on the type of raw material.

By applying the adsorption purification process using an adsorbent, not only an alkylene carbonate, which is the main synthesis target material in the present invention, be secured with high purity, but also the content of other impurities can be significantly reduced.

Adsorption purification conditions by the adsorbent are not particularly limited, and various well-known adsorption purification conditions can be used without limitation. However, as an example, the addition amount of the adsorbent may be 40% by weight to 60% by weight relative to the polycarbonate-based resin, and the adsorption time may be 0.1 hour to 5 hours. As for the adsorption method, stirring adsorption or a Lab adsorption tower can be used.

Meanwhile, the method for preparing the recycled cyclic carbonate composition of the other embodiment may comprise a step of recovering an alkylene carbonate from the depolymerized solution from which the aromatic diol compound, the organic solvent, and the alkylene glycol have been removed.

The step of recovering an alkylene carbonate from the depolymerized solution from which the aromatic diol compound, the organic solvent, and the alkylene glycol have been removed may comprise a step of distilling the depolymerized solution from which the aromatic diol compound, the organic solvent, and the alkylene glycol have been removed.

In the step of recovering an alkylene carbonate from the depolymerized solution from which the aromatic diol compound, the organic solvent, and the alkylene glycol have been removed, the organic solvent remaining in the depolymerized solution from which the aromatic diol compound, the organic solvent, and the alkylene glycol have been removed can be removed through distillation.

The distillation temperature is adjusted to a temperature lower than the boiling point of the alkylene carbonate and higher than the boiling point of the organic solvent, so that the alkylene carbonate remains without being distilled, and the organic solvent can be removed by distillation. Other distillation conditions are not particularly limited, and various well-known distillation conditions, methods, and equipment can be applied without limitation.

Specifically, the step of distilling the organic solvent may proceed at a temperature of 40°C to 80°C, or 40°C to 70°C, or 40°C to 60°C, or 40°C to 50°C and under a pressure of 100 psi to 200 psi, or 120 psi to 180 psi, or 140 psi to 160 psi.

### [Advantageous Effects]

According to the present invention, there can be provided a recycled cyclic carbonate composition and a preparation method of the same, in which a cyclic carbonate compound recovered through recycling by chemical decomposition of a polycarbonate-based resin can be secured with high purity.

### [DETAILED DESCRIPTION OF THE EMBODIMENTS]

Hereinafter, the present invention will be explained in detail with reference to the following examples. However, these examples are for illustrative purposes only, and the scope of the present invention is not limited thereto.

### <EXAMPLE>

### Example 1

166.67 g of Methylene chloride and 30 g of polycarbonate were added to a 500 ml three-neck flask, and then stirred.

Then, 16.37 g of ethylene glycol and 0.169 g of sodium hydroxide (NaOH) as a catalyst were added thereto, and subjected to a depolymerization reaction at 100°C for 3 hours.

When the depolymerization reaction was completed, water was added to the depolymerization reaction product to crystallize bisphenol A, which was then filtered under reduced pressure to remove bisphenol A. The resulting filtrate was then distilled at 40°C and 150 psi to remove methylene chloride, and then treated with charcoal to remove phenolic impurities.

The resulting filtrate was then separated into layers using water and methylene chloride, the water layer containing ethylene glycol was removed to obtain a methylene chloride layer containing ethylene carbonate. The resulting methylene chloride solution was distilled at 40°C and 150 psi to remove methylene chloride and obtain ethylene carbonate. Thereby, a recycled cyclic carbonate composition was prepared.

### Example 2

A recycled cyclic carbonate was prepared in the same manner as in Example 1, except that acid clay was used instead of charcoal.

### Example 3

A recycled cyclic carbonate was prepared in the same manner as in Example 1, except that the amount of ethylene glycol used was changed to 19.65g.

### Example 4

A recycled cyclic carbonate was prepared in the same manner as in Example 1, except that the amount of ethylene glycol used was changed to 22.92g.

### Example 5

A recycled cyclic carbonate was prepared in the same manner as in Example 1, except that the amount of sodium hydroxide (NaOH) used was changed to 0.127g.

### Example 6

A recycled cyclic carbonate was prepared in the same manner as in Example 1, except that 26.76g of propylene glycol was used instead of ethylene glycol.

### Example 7

Propylene carbonate was obtained in the same manner as in Example 1, except that 20.07g of propylene glycol was used instead of ethylene glycol, thereby preparing a recycled cyclic carbonate.

### <COMPARATIVE EXAMPLE>

### Comparative Example 1

60 ml of Methylene chloride, 30 ml of ethylene glycol, and 0.6 g of sodium hydroxide(NaOH) were added to a 250 ml three-neck flask, and then stirred.

Then, 30 g of waste polycarbonate was added thereto, and the mixture was stirred at 40°C for 5 hours.

When the reaction was completed, ethylene glycol was distilled off, and then toluene was added thereto to separate bisphenol A and obtain ethylene carbonate. Thereby, a recycled cyclic carbonate composition was prepared.

### Comparative Example 2

60 ml of Toluene, 30 ml of ethylene glycol, and 0.6 g of sodium hydroxide(NaOH) were added to a 250 ml three-neck flask, and then stirred.

Then, 30 g of waste polycarbonate was added thereto, and the mixture was stirred at 60°C for 5 hours.

When the reaction was completed, ethylene glycol was distilled off, and then toluene was added thereto to separate bisphenol A and obtain ethylene carbonate. Thereby, a recycled cyclic carbonate composition was prepared.

### Comparative Example 3

120 g of phenol and 40 g of polycarbonate were added to a 500 ml three-neck flask, and then stirred.

Then, 17.5 g of ethylene glycol and 1 g of sodium hydroxide (NaOH) as a catalyst were added thereto, and subjected to a depolymerization reaction at 80°C for 5 hours.

When the depolymerization reaction was completed, 100 g of toluene was added to the depolymerization reaction product, and the mixture was neutralized using hydrochloric acid, and distilled at 120°C and 150 psi to remove phenol, and then treated with charcoal to remove phenolic impurities.

The resulting filtrate was then separated into layers using water and methylene chloride, the water layer containing ethylene glycol was removed to obtain a methylene chloride layer containing ethylene carbonate. The resulting methylene chloride solution was distilled at 40°C and 150 psi to remove methylene chloride and obtain ethylene carbonate. Thereby, a recycled cyclic carbonate composition was prepared.

### <Experimental Example>

The physical properties of the recycled cyclic carbonate compositions obtained in Examples and Comparative Examples were measured by the following methods, and the results are shown in Tables 1 to 3 below.

### 1. NMR analysis

The recycled cyclic carbonate composition was collected as a sample, diluted with DMSO-d6 to a concentration of 10 mg/ml, and then the integral value for the NMR peak detected through ¹H NMR equipment was measured, and the mass ratios of ethylene carbonate(EC), ethylene glycol(EG), bisphenol A(BPA), 4-tert-butylphenol(PTBP), and monohydroxyethyl-bisphenol A(MHE-BPA) contained in the sample was determined through the following Equations 1 to 5.

Specifically, ethylene carbonate (EC) targeted at a peak at 4.48 ppm (s, 2CH₂), ethylene glycol(EG) targeted at a peak at 3.42 ppm (d, 2CH₂), bisphenol A (BPA) targeted at a peak at 6.67 ppm (d, 4CH), 4-tert-butylphenol(PTBP) targeted at a peak at 6.60 ppm (d, 2CH), and monohydroxyethyl-bisphenol A(MHE-BPA) targeted at a peak at 6.85 ppm (d, 2CH). Ethylene carbonate(EC) mass ratio (mass%) = (Mol.eqEC X MWEC)/[(Mol.eqPTBP X MWPTBP) + (Mol.eqMHE-BPA X MWMHE-BPA) + (Mol.eqBPA X MWBPA) + (Mol.eqEG X MWEG) + (Mol.eqEC X MWEC)] X 100
wherein, in Equation 1, MW_{EC} is the molecular weight of ethylene carbonate(EC), MW_{EG} is the molecular weight of ethylene glycol(EG), MW_{BPA} is the molecular weight of bisphenol A (BPA), MW_{PTBP} is the molecular weight of 4-tert-butylphenol(PTBP), MW_{MHE-BPA} is the molecular weight of monohydroxyethyl-bisphenol A(MHE-BPA),
Mol.eq_{EC} is I_{EC}/H_{EC},
I_{EC} is (S_{EC})/[(S_{PTBP})+(S_{MHE-BPA})+(S_{BPA})+(S_{EG})+(S_{EC})],
S_{EC} is the peak integral value at 4.48 ppm (s, 2CH₂) of ethylene carbonate(EC), S_{EG} is the peak integral value at 3.42 ppm (d, 2CH₂) of ethylene glycol(EG), S_{BPA} is the peak integral value at 6.67 ppm (d, 4CH) of bisphenol A(BPA), S_{PTBP} is the peak integral value at 6.60 ppm (d, 2CH) of 4-tert-butylphenol(PTBP), S_{MHE-BPA} is the peak integral value at 6.85 ppm (d, 2CH) of monohydroxyethyl-bisphenol A(MHE-BPA), H_{EC} is the number of H in ethylene carbonate(EC), Mol.eq_{EG} is I_{EG}/H_{EG},
I_{EG} is (S_{EG})/[(S_{PTBP})+(S_{MHE-BPA})+(S_{BPA})+(S_{EG})+(S_{EC})],
H_{EG} is the number of H in ethylene glycol(EG),
Mol.eq_{BPA} is I_{BPA}/H_{BPA},
I_{BPA} is (S_{BPA})/[(S_{PTBP})+(S_{MHE-BPA})+(S_{BPA})+(S_{EG})+(S_{EC})],
H_{BPA} is the number of H in bisphenol A(BPA),
Mol.eq_{PTBP} is I_{PTBP}/H_{PTBP},
I_{PTBP} is (S_{PTBP})/[(S_{PTBP})+(S_{MHE-BPA})+(S_{BPA})+(S_{EG})+(S_{EC})],
H_{PTBP}i s the number of H in 4-tert-butylphenol(PTBP),
Mol.eq_{MHE-BPA} i s I_{MHE-BPA}/H_{MHE-BPA},
I_{MHE-BPA} is (S_{MHE-BPA})/[(S_{PTBP})+(S_{MHE-BPA})+(S_{BPA})+(S_{EG})+(S_{EC})],
H_{MHE-BPA} is the number of H in monohydroxyethyl-bisphenol A(MHE-BPA). EG mass ratio (mass%) = (Mol.eqEG X MWEG)/[(Mol.eqPTBP X MWPTBP) + (Mol.eqMHE-BPA X MWMHE-BPA) + (Mol.eqBPA X MWBPA) + (Mol.eqEG X MWEG) + (Mol.eqEC X MWEC)] X 100 BPA mass ratio (mass%) = (Mol.eqBPA X MWBPA)/[(Mol.eqPTBP × MWPTBP) + (Mol.eqMHE-BPA X MWMHE-BPA) + (Mol.eqBPA X MWBPA) + (Mol.eqEG X MWEG) + (Mol.eqEC X MWEC)] X 100 PTBP mass ratio (mass%) = (Mol.eqPTBP X MWPTBP)/[(Mol.eqPTBP X MWPTBP) + (Mol.eqMHE-BPA X MWMHE-BPA) + (Mol.eqBPA X MWBPA) + (Mol.eqEG X MWEG) + (Mol.eqEC X MWEC)] X 100 MHE-BPA mass ratio (mass%) = (Mol.eqMHE-BPA X MWMHE-BPA)/[(Mol.eqPTBP X MWPTBP) + (Mol.eqMHE-BPA X MWMHE-BPA) + (Mol.eqBPA X MWBPA) + (Mol.eqEG X MWEG) + (Mol.eqEC X MWEC)] X 100

**[Table 1]**

| Measurement results of Experimental Example 1 (unit: mass%) | | | | | |
|---|---|---|---|---|---|
| Category | EC | EG | BPA | PTBP | MHE-BPA |
| Example 1 | 98.46 | 0.38 | 0.94 | 0.22 | 0.00 |
| Example 2 | 95.78 | 0.42 | 2.83 | 0.51 | 0.47 |
| Example 3 | 96.10 | 2.49 | 0.04 | 0.56 | 0.82 |
| Example 4 | 95.11 | 2.17 | 1.38 | 0.47 | 0.87 |
| Example 5 | 95.50 | 1.95 | 1.24 | 0.48 | 0.83 |
| Example 6 | 95.20 | 0.48 | 2.87 | 0.54 | 0.91 |
| Example 7 | 95.14 | 0.51 | 2.97 | 0.57 | 0.81 |
| Comparative Example 1 | 92.96 | 0.05 | 4.23 | 1.00 | 1.77 |
| Comparative Example 2 | 94.20 | 0.49 | 2.84 | 1.07 | 1.39 |
| Comparative Example 3 | 92.57 | 1.92 | 3.21 | 1.07 | 1.23 |

As shown in Table 1, it could be confirmed that in the recycled cyclic carbonat1e compositions obtained in Examples, the content of ethylene carbonate(EC) is high and the content of phenolic impurities such as bisphenol A(BPA), 4-tert-butylphenol(PTBP) and monohydroxyethyl-bisphenol A(MHE-BPA) are reduced as compared to Comparative Examples.

### 2. GC-MS analysis

The recycled cyclic carbonate composition was collected as a sample, diluted with methanol to a concentration of 10 mg/ml, and then a gas chromatography mass spectrometry (GC-MS) spectrum was determined using GC/MS equipment under the following conditions, and the peak area ratios of ethylene carbonate(EC), ethylene glycol(EG), bisphenol A(BPA), 4-tert-butylphenol(PTBP), and monohydroxyethyl-bisphenol A(MHE-BPA) contained in the sample was determined.

Specifically, ethylene carbonate (EC) targeted at a peak at a retention time of 8 minutes to 11 minutes, bisphenol A (BPA) targeted at a peak at a retention time of 24 minutes to 26 minutes, 4-tert-butylphenol (PTBP) targeted at a peak at a retention time of 14 minutes to 16 minutes, and monohydroxyethyl-bisphenol A (MHE-BPA) targeted at a peak at a retention time of 26 minutes to 28 minutes.

### <Gas chromatography (GC) conditions>

① Column: HP-SMS(L:30m, ID:0.25mm, film:0.25µm)
② Injection volume: 0.2*µ*ℓ
(3) Inlet, Temp.: 300 °C, spilt ratio: 20:1
④ Column flow: (He) 1ml/min
⑤ Oven temp.: 50°C/5min-10°C/min-320°C/15min (Total 47min)
⑥ Detector Temp.:300°C
⑦ GC Model: Agilent 7890

**[Table 2]**

| Measurement results of Experimental Example 2 (unit: area%) | | | | |
|---|---|---|---|---|
| Category | EC | BPA | PTBP | MHE-BPA |
| Example 1 | 97.84 | 0.41 | 1.66 | 0 |
| Example 2 | 88.92 | 2.62 | 8.39 | 0.07 |
| Example 3 | 96.21 | 0.67 | 3.12 | 0 |
| Example 4 | 95.22 | 0.85 | 2.62 | 0.02 |
| Example 5 | 95.61 | 0.77 | 2.67 | 0.03 |
| Example 6 | 95.31 | 1.78 | 3.01 | 0 |
| Example 7 | 95.25 | 1.84 | 3.18 | 0.01 |
| Comparative Example 1 | 85.24 | 2.62 | 11.39 | 0.07 |
| Comparative Example 2 | 74.67 | 7.96 | 17.09 | 0.28 |
| Comparative Example 3 | 86.39 | 4.31 | 9.21 | 0.09 |

As shown in Table 2, it could be confirmed that in the recycled cyclic carbonate composition obtained in Examples, the proportion of ethylene carbonate (EC) is high, and the proportion of phenolic impurities such as bisphenol A (BPA), 4-tert-butylphenol (PTBP), and monohydroxyethyl-bisphenol A (MHE-BPA) is reduced as compared to Comparative Examples.

### 3. Ion analysis

The recycled cyclic carbonate composition was collected as a sample, and analyzed by inductively coupled plasma mass spectrometer(ICP-MS) and ion chromatography(IC) under the following conditions. The weight ratio (unit: ppm) of ions contained in the sample was measured based on 1 g of the sample.

### <Inductively coupled plasma mass spectrometer (ICP-MS) conditions>

① Analysis conditions (aqueous sample, No gas mode)
② RF power(W): 1600
③ RF Matching (V): 1.6
④ Sample Depth (nm) 8
⑤ Carrier Gas (L/min): 0.7
⑥ Option Gas (%): 0
⑦ S/C Temp (°C): 2
⑧ Makeup Gas (L/min): 0.52

### <Ion chromatography (IC) conditions>

① Main Column: IonPac AS 18 analytical (4X250 mm)
② Guard Column: Ion AG18 guard (4X50 mm)
③ Type of Eluent: KOH (30.5mM)
④ Eluent flow rate: 1mL/min
⑤ Detector: Suppressed Conductivity Detector
⑥ SRS Current: 76mA
⑦ Injection volume: 20*µ*ℓ
⑧ Isocratic/Gradient condition: isocratic

**[Table 3]**

| Measurement results of Experimental Example 3 (unit: ppm) | | | | | | | | | | |
|---|---|---|---|---|---|---|---|---|---|---|
| Iο n | Exampl e 1 | Exampl e 2 | Exampl e 3 | Exampl e 4 | Exampl e 5 | Exampl e 6 | Exampl e 7 | Comparativ e Example 1 | Comparativ e Example 2 | Comparativ e Example 3 |
| Li | less than 0.1 | less than 0.1 | less than 0.1 | less than 0.1 | less than 0.1 | less than 0.1 | less than 0.1 | less than 0.1 | less than 0.1 | less than 0.1 |
| Na | 0.1 | 0.2 | 0.1 | 0.1 | 0.1 | 0.2 | 0.3 | 0.7 | 0.9 | 0.4 |
| M g | 0.1 | 0.1 | 0.1 | 0.1 | 0.1 | 0.1 | 0.1 | 0.1 | 0.1 | 0.1 |
| Al | less than 0.1 | 0.1 | less than 0.1 | 0.1 | 0.1 | less than 0.1 | 0.1 | 0.1 | 0.1 | 0.1 |
| K | 0.1 | 0.2 | 0.1 | 0.1 | 0.2 | 0.1 | 0.3 | 0.8 | 0.5 | 0.3 |
| Ca | 0.3 | 0.3 | 0.1 | 0.3 | 0.1 | 0.1 | 0.3 | 0.7 | 0.5 | 0.4 |
| Ti | less than 0.1 | 0.1 | less than 0.1 | less than 0.1 | less than 0.1 | less than 0.1 | 0.1 | 0.1 | 0.1 | 0.1 |
| V | less than 0.1 | less than 0.1 | less than 0.1 | less than 0.1 | less than 0.1 | less than 0.1 | less than 0.1 | less than 0.1 | less than 0.1 | less than 0.1 |
| Cr | less than 0.1 | less than 0.1 | less than 0.1 | less than 0.1 | less than 0.1 | less than 0.1 | less than 0.1 | less than 0.1 | less than 0.1 | less than 0.1 |
| M n | less than 0.1 | less than 0.1 | less than 0.1 | less than 0.1 | less than 0.1 | less than 0.1 | less than 0.1 | less than 0.1 | less than 0.1 | less than 0.1 |
| Fe | less than 0.1 | 0.5 | less than 0.1 | less than 0.1 | less than 0.1 | less than 0.1 | 0.2 | 0.5 | 0.8 | 0.4 |
| Co | less than 0.1 | less than 0.1 | less than 0.1 | less than 0.1 | less than 0.1 | less than 0.1 | less than 0.1 | less than 0.1 | less than 0.1 | less than 0.1 |
| Ni | less than 0.1 | less than 0.1 | less than 0.1 | less than 0.1 | less than 0.1 | less than 0.1 | less than 0.1 | less than 0.1 | less than 0.1 | less than 0.1 |
| Cu | less than 0.1 | less than 0.1 | less than 0.1 | less than 0.1 | less than 0.1 | less than 0.1 | less than 0.1 | less than 0.1 | less than 0.1 | less than 0.1 |
| Zn | less than 0.1 | 0.1 | less than 0.1 | less than 0.1 | less than 0.1 | less than 0.1 | 0.1 | 0.2 | 0.3 | 0.1 |
| Cl | 10 | 10 | 10 | 13 | 10 | 11 | 20 | 138 | 278 | 23 |
| Br | less than 2 | less than 2 | less than 2 | less than 2 | less than 2 | less than 2 | less than 2 | less than 2 | less than 2 | less than 2 |

As shown in Table 3, it could be confirmed that the recycled cyclic carbonate compositions obtained in Examples is reduced in terms of the individual ion content or the sum of all ion contents as compared to the Comparative Examples.

## Claims

1. A recycled cyclic carbonate composition, comprising an alkylene carbonate,
wherein the recycled cyclic carbonate composition further comprises a phenolic compound whose mass ratio measured by ¹H NMR is 5 mass% or less relative to a total mass of the composition, and
wherein the recycled cyclic carbonate composition is a recovered product from a polycarbonate-based resin.

2. The recycled cyclic carbonate composition according to claim 1, wherein:
a peak area ratio of the alkylene carbonate measured by GC-MS is 87 area% to 99.99 area% relative to a total peak area.

3. The recycled cyclic carbonate composition according to claim 1, wherein:
the mass ratio of the alkylene carbonate measured by ¹H NMR is 95 mass% to 98.5 mass% relative to the total mass of the composition.

4. The recycled cyclic carbonate composition according to claim 1, wherein:
in the recycled cyclic carbonate composition, the mass ratio of the phenolic compound relative to 100 parts by mass of the alkylene carbonate is 0.01 parts by mass to 5 parts by mass.

5. The recycled cyclic carbonate composition according to claim 1, wherein:
the phenolic compound comprises one or more compounds selected from the group consisting of bisphenol A, 4-tert-butylphenol, and monohydroxyethyl-bisphenol A.

6. The recycled cyclic carbonate composition according to claim 1, wherein:
the peak area ratio of the phenolic compound measured by GC-MS is 0.01 area% to 12 area% relative to a total peak area.

7. The recycled cyclic carbonate composition according to claim 1, further comprising an alkylene glycol,
the mass ratio of the alkylene glycol measured by ¹H NMR is 0.1 mass% to 3 mass% relative to the total mass of the composition.

8. The recycled cyclic carbonate composition according to claim 7, wherein:
in the recycled cyclic carbonate composition, the mass ratio of the alkylene glycol relative to 100 parts by mass of the alkylene carbonate is 0.1 parts by mass to 3 parts by mass.

9. The recycled cyclic carbonate composition according to claim 7, wherein:
the mass ratio of the phenolic compound relative to 100 parts by mass of the alkylene glycol is 30 to 1000 parts by mass.

10. The recycled cyclic carbonate composition according to claim 1, wherein:
in the recycled cyclic carbonate composition, a calcium ion weight ratio measured using ion chromatography is 0.05 ppm to 0.4 ppm based on 1 g of the composition.

11. The recycled cyclic carbonate composition according to claim 1, wherein:
in the recycled cyclic carbonate composition, the chloride ion weight ratio measured using ion chromatography is 5.1 ppm to 20 ppm based on 1 g of the composition.

12. The recycled cyclic carbonate composition according to claim 1, wherein:
in the recycled cyclic carbonate composition, the total ion concentration measured using ion chromatography is 300 ppm or less based on 1 g of the composition.

13. A method for preparing the recycled cyclic carbonate composition of claim 1, the method comprising the steps of:
adding a polycarbonate-based resin to an organic solvent to prepare a mixed solution;
adding an alkylene glycol and an inorganic base catalyst to the mixed solution to depolymerize the polycarbonate-based resin;
removing an aromatic diol compound, an organic solvent, and an alkylene glycol from the depolymerized solution; and
recovering an alkylene carbonate from the depolymerized solution from which the aromatic diol compound, the organic solvent, and the alkylene glycol have been removed.

14. The method for preparing the recycled cyclic carbonate composition according to claim 13, wherein:
the inorganic base catalyst comprises sodium hydroxide or potassium hydroxide.

15. The method for preparing the recycled cyclic carbonate composition according to claim 13, wherein:
the organic solvent comprises one or more solvents selected from the group consisting of tetrahydrofuran, methylene chloride, chloroform, dimethyl carbonate, ethylmethyl carbonate, diethyl carbonate and dipropylcarbonate.

16. The method for preparing the recycled cyclic carbonate composition according to claim 13, wherein:
the adding an alkylene glycol and an inorganic base catalyst to the mixed solution to depolymerize the polycarbonate-based resin is stirring at 20°C to 100°C for 1 hour to 30 hours.

17. The method for preparing the recycled cyclic carbonate composition according to claim 13, wherein:
the removing an aromatic diol compound, an organic solvent, and an alkylene glycol from the depolymerized solution comprises the steps of:
removing the aromatic diol compound from the depolymerized solution;
removing the organic solvent from the depolymerized solution; and
removing the alkylene glycol from the depolymerized solution.

18. The method for preparing the recycled cyclic carbonate composition according to claim 17, wherein:
the removing the aromatic diol compound from the depolymerized solution comprises the steps of:
crystallizing the aromatic diol compound formed by the depolymerization; and
filtering the aromatic diol compound crystals.

19. The method for preparing the recycled cyclic carbonate composition according to claim 17, wherein:
the removing the alkylene glycol from the depolymerized solution comprises,
adding the depolymerized solution to a solution separated into a water layer and an organic solvent layer and removing alkylene glycol dissolved in the water layer.

20. The method for preparing the recycled cyclic carbonate composition according to claim 13, wherein:
the recovering an alkylene carbonate from the depolymerized solution from which the aromatic diol compound, the organic solvent, and the alkylene glycol have been removed comprises,
distilling the depolymerized solution from which the aromatic diol compound, the organic solvent, and the alkylene glycol have been removed.
